(19) Europäisches Patentamt
European Patent Office
Office européen des brevets

(11) **EP 1 891 935 A1**

(12) **EUROPÄISCHE PATENTANMELDUNG**

(43) Veröffentlichungstag:
**27.02.2008 Patentblatt 2008/09**

(51) Int Cl.:
*A61K 8/86* *(2006.01)*    *A61K 8/31* *(2006.01)*
*A61K 8/37* *(2006.01)*    *A61K 8/34* *(2006.01)*
*A61K 8/02* *(2006.01)*    *A61Q 19/00* *(2006.01)*
*A61K 8/06* *(2006.01)*    *A61K 9/107* *(2006.01)*

(21) Anmeldenummer: 06016953.9

(22) Anmeldetag: **14.08.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **Cognis IP Management GmbH**
**40589 Düsseldorf (DE)**

(72) Erfinder:
• **Strauss, Gabriele**
**40589 Düsseldorf (DE)**
• **Kawa, Rolf**
**40789 Monheim (DE)**
• **Schulte, Petra**
**41466 Neuss (DE)**
• **Stork, Anja**
**50733 Köln (DE)**

(54) **Emulsionskonzentrat**

(57) Die vorliegende Erfindung betrifft ein Emulsionskonzentrat mit einem Gehalt an wasserunlöslichen Ölkomponenten (A), hydrophilen nichtionischen Emulgatoren (B), lipophilen Coemulgatoren (C), Polyolen (D) und Wasser, welches weniger als 50 Gew.-% wasserunlösliche Ölkomponente (A) enthält - bezogen auf das Gesamtgewicht des Konzentrats - und die Komponenten (A), (B) und (C) und (D) im Gewichtsverhältnis A : B : C : D =1: (0,25 - 0,6) : (0,25 - 0,6) : (0,45 - 0,65) enthält. Die Erfindung betrifft weiterhin die Verwendung solcher Emulsionskonzentrate, sowie Zubereitungen und Papier-, Vlies- oder Gewebe-Produkt zur Körperpflege und -reinigung, welche wasserunlösliche Ölkomponenten (A), hydrophile nichtionische Emulgatoren (B), lipophile Coemulgatoren (C), Polyole (D) im Gewichtsverhältnis A : B : C : D =1; (0,25 - 0,6) : (0,25 - 0,6) : (0,45 - 0,65) enthalten.

**EP 1 891 935 A1**

**Beschreibung**

Die Erfindung betrifft ein bei Normaltemperatur fließ- und pumpfähiges Emulsionskonzentrat, sowie dessen Verwendung zur Herstellung von kosmetischen und pharmazeutischen Zubereitungen.

Die Herstellung emulsionsförmiger Zubereitungen erfordert normalerweise einen erheblichen apparativen Aufwand, da die disperse Phase durch Erwärmen verflüssigt und unter Anwendung von Scherenergie in der kontinuierlichen Phase dispergiert werden muss. Es hat schon verschiedene Versuche gegeben, Emulsionskonzentrate herzustellen, die ohne Wärmezufuhr und ohne Scherarbeit mit der kontinuierlichen Phase verdünnt werden können.

So sind z.B. aus EP 0 723 432 B1 fließfähige Emulsionskonzentrate bekannt, welche bei Normaltemperatur fließfähig und pumpbar sind und sich mit Wasser und Ölkomponenten und ggf. weiteren Zusätzen ohne Wärmezufuhr und unter Aufwendung nur geringer Rührenergie zu emulsionsförmigen Zubereitungen weiterverarbeiten lassen.

US 2006/0013783 A1 beschreibt feinteilige kosmetische oder dermatologische O/W Emulsionen.

Nachteilig an den in EP 0 723 432 B1 beschriebenen Emulsionskonzentraten ist ihre geringe Lagerstabilität bei erhöhten Temperaturen, insbesondere bei Temperaturen von über 40 °C sowie bei längeren Lagerzeiten. Unter diesen Bedingungen kommt es zu einer Separation.der Phasen. Weiterhin besteht das Bedürfnis nach Emulsionskonzentraten, die einen möglichst geringen wässrigen Anteil enthalten, da sich dadurch Transport- und Lagerkosten senken lassen.

Überraschenderweise wurde gefunden, dass die Emulsionskonzentrate der vorliegenden Erfindung diese Aufgaben lösen.

Gegenstand der Erfindung ist daher ein Emulsionskonzentrat mit einem Gehalt-an wasserunlöslichen Ölkomponenten (A), hydrophilen nichtionischen Emulgatoren (B), lipophilen Coemulgatoren (C), Polyolen (D) und Wasser welches

> a. weniger als 50 Gew.-% wasserunlösliche Ölkomponente (A) enthält

> -   bezogen auf das Gesamtgewicht des Konzentrats -

> b. die Komponenten (A), (B) und (C) und (D) im Gewichtsverhältnis A : B : C : D = 1: (0,25

> -   0,6) : (0,25 - 0,6) : (0,45 - 0,65) enthält.

Das erfindungsgemäße Emulsionskonzentrat ist bevorzugt bei 20 °C fließfähig und pumpbar. Als fließfähig oder pumpfähig werden dabei solche Emulsionskonzentrate bezeichnet, deren Viskosität bei 20°C unterhalb 20 Pa·s, gemessen mit einem Brookfield Rotationsviskosimeter (Typ RVF, Spindel 4, 10 UpM), liegt.

Wasserunlösliche Komponenten (A)

Als wasserunlösliche Ölkomponenten (A) eignen sich alle bei 30°C flüssigen Fettstoffe oder Fettstoffgemische, d.h. auch Gemische aus flüssigen und darin gelösten, festen Fettstoffen oder Paraffinen, solange diese Gemische bei 30°C flüssig sind bzw, deren Viskosität (20°C) unter 20 Pa·s liegt.

Bevorzugt eignen sich als Ölkomponenten (A) die bei 30°C flüssigen Kohlenwasserstoffe, Dialkylether, Fettsäureester mit 12 - 44 C-Atomen, Dialkylcarbonate, Guerbetalkohole und Siliconöle oder deren Mischungen

In einer bevorzugten Ausführungsform der Erfindung weisen mindestens 60 Gew.-% der wasserunlöslichen Ölkomponente (A) eine mittlere Polarität von größer gleich 20 mN/m und kleiner gleich 30 mN/m auf. Besonders bevorzugt weisen mindestens 70 Gew.-%, insbesondere mindestens 80 Gew.-% der wasserunlöslichen Ölkomponente (A) eine mittlere Polarität von größer gleich 20 mN/m und kleiner gleich 30 mN/m auf. Die Gew.% sind bezogen auf die Gesamtmenge an wasserunlöslicher Komponente (A).

Die Polarität der wasserunlöslichen Komponente (A) wird über die Grenzflächenspannung angegeben.

Die Grenzflächenspannung ist die diejenige Kraft, die an einer gedachten, in der Grenzfläche zwischen zwei Phasen befindlichen Linie der Länge von einem Meter wirkt. Die physikalische Einheit für die Grenzflächenspannung errechnet sich klassisch nach der Beziehung Kraft/Länge und wird gewöhnlich in mN/m (Millinewton geteilt durch Meter) wiedergegeben. Sie hat ein positives Vorzeichen, wenn sie das Bestreben hat, die Grenzfläche zu verkleinern, im umgekehrten Fall hat sie ein negatives Vorzeichen. Die Grenzflächenspannung kann beispielsweise bestimmt werden nach der ASTM Methode D971-99a (reapproved 2004).

Durch den hohen Anteil an wasserunlöslicher Komponente (A) dieser Polarität, werden besonders stabile Emulsionskonzentrate erhalten.

Die wasserunlösliche Komponenten (A) kann Ölkomponenten, Fette, Wachse sowie beliebige Mischungen daraus enthalten.

In einer Ausführungsform der Erfindung enthält die wasserunlösliche Komponenten (A) mindestens ein Öl.

Unter dem Begriff "Öle" (synonym verwendet: Ölkomponente) werden wasserunlösliche, bei 30°C flüssige, organische Verbindungen mit relativ niedrigem Dampfdruck bezeichnet. Das gemeinsame Merkmal der Öle ist nicht ihre übereinstimmende chemische Konstitution, sondere ihre ähnliche physikalische Konsistenz.

Als Ölkomponenten sind beispielsweise die nachstehend genannten Verbindungsklassen geeignet, soweit diese bei 30°C flüssig sind. So z.B. Guerbetalkohole auf Basis von Fettalkoholen mit 6 bis 18, vorzugsweise 8 bis 10 Kohlenstoffatomen (z. B. Eutanol® G), Ester von linearen $C_6$-$C_{22}$-Fettsäuren mit linearen oder verzweigten $C_6$-$C_{22}$-Feftalkoholen bzw. Ester von verzweigten $C_6$-$C_{13}$-Carbonsäuren mit linearen oder verzweigten $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat, Erucylerucat und Hexyldecylstearat (Eutanol® G 16 S). Daneben eignen sich Ester von linearen $C_6$-$C_{22}$-Fettsäuren mit verzweigten Alkoholen, insbesondere 2-Ethylhexanol, Ester von $C_3$-$C_{38}$-Alkylhydroxycarbonsäuren mit linearen oder verzweigten $C_6$-$C_{22}$-Fettalkoholen - insbesondere Dioctylmalat-, Ester von linearen und/oder verzweigten Fettsäuren mit mehrwertigen Alkoholen (wie z.B. Propylenglycol,- Dimerdiol oder Trimertriol) und/oder Guerbetalkoholen, Triglyceride auf Basis $C_6$-$C_{10}$-Fettsäuren, flüssige Mono-/Di-/Triglycerid-mischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane wie z.B. 1,3-Dialkylcyclohexane, lineare und verzweigte $C_6$-$C_{22}$-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen (Hydagen® HSP, Sovermol® 750, Sovermol® 1102), Siliconöle (Cyclomethicone, Siliciummethicontypen u.a. und/oder aliphatische bzw. naphthenische Kohlenwasserstoffe, wie z.B. wie Mineralöl, Vaseline, Petrolatum, Squalan, Squalen oder Dialkylcyclohexane.

Als Siliconöle geeignet sind neben Dimethylpolysiloxanen, Methylphenylpolysiloxanen und cyclischen Siliconen auch amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und Siliciumdioxid oder hydrierten Silicaten handelt.

Als Ölkörper eignen sich auch Polycarbonate, wie beispielsweise in WO 031041676 beschrieben, auf die hier ausdrücklich Bezug genommen wird.

Als Polycarbonate besonders geeignet ist das unter, der INCI Bezeichnung Hydrogenated Dimer Dilinoleyl / Dimethylcarbonate Copolymer, welches als Handelsprodukt Cosmedia® DC von Cognis Deutschland GmbH & Co. KG erhältlich ist.

Dialkylether, Dialkylcarbonate, Triglycerid-Mischungen und Ester aus C6-C22-Fettsäuren und C6-C22 Fettalkoholen, Polycarbonate bzw. ein Gemisch dieser Substanzen sind erfindungsgemäß als Ölkörper besonders gut geeignet. Die Dialkylcarbonate und Dialkylether können symmetrisch oder unsymmetrisch, verzweigt oder unverzweigt, gesättigt oder ungesättigt sein-und lassen sich nach Reaktionen, die aus dem Stand der Technik hinlänglich bekannt sind, herstellen.

Erfindungsgemäß einsetzbar sind u.a. auch Kohlenwasserstoffe, vorzugsweise mit einer Kettenlänge 8 bis 40 C-Atomen. Sie können verzweigt oder unverzweigt sein, gesättigt oder ungesättigt. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.% verzweigte Alkane bezogen auf die Gesamtmenge der Alkane enthält. Vorzugsweise handelt es sich um verzweigte, gesättigte Alkane. Besonders gut geeignet sind Gemische aus Alkanen, welche mehr als 1 Gew.-% 5,8-Diethyldodecan und/oder mehr als 1 Gew.-% Didecen enthalten.

In einer Ausführungsform der Erfindung, enthält die wasserunlösliche Komponenten (A) Phase mindestens ein Wachs.

Unter dem Begriff Wachs (synonym verwendet: Wachskomponente) werden üblicherweise alle natürlichen oder künstlich gewonnenen Stoffe und Stoffgemische mit folgenden Eigenschaften verstanden: sie sind von fester bis brüchig harter Konsistenz, grob bis feinteilig, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

In einer bevorzugten Ausführungsform enthält die wasserunlösliche Komponente (A) mindestens ein **hydrophiles Wachs.** Hydrophile Wachse sind gekennzeichnet durch einen Schmelzpunkt von über 30°C und das Vorhandensein mindestens einer funktionellen OH-Gruppe. In Abgrenzung zu den Emulgatoren, weisen hydrophile Wachse einen HLB-Wert von kleiner 10 auf.

In einer bevorzugten Ausführungsform enthält die wasserunlösliche Komponente (A) als hydrophiles Wachs mindestens einen Partialester von C12 bis C 22 Fettsäuren mit mehrwertigen Alkoholen.

Die mehrwertigen Alkohole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind:

- Glycerin
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1,000 Dalton;
- technische Oligoglyceringemische mit einem Eigenkondensationsgrad von 1,5 bis 10 wie etwa technische Diglyceringemische mit einem Diglyceringehalt von 40 bis 50 Gew.%;
- Methyolverbindungen, wie insbesondere Trimethylolethan, Trimethylolpropan, Trimethylolbutan, Pentaerythrit und Dipentaerythrit;
- kurzkettige Alkyglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Typische Beispiele für geeignete Partialglyceride sind Mono- und/oder Diglyceride von C 12 bis C 22 Fettsäuren mit Glycerin sowie deren technische Gemische, sofern sie einen Schmelzpunkt von >30°C aufweisen. Beispielsweise seien genannt langkettige Hydroxyfettsäuremonoglyceride, langkettige Hydroxyfettsäurediglyceride, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid.

Typische Beispiele für geeignete Partialgylceride sind Mono- und/oder Diglyceride von Dicarbonsäuren mit 4 bis 8 C-Atomen mit Glycerin sowie deren technische Gemische, sofern sie einen Schmelzpunkt von >30°C aufweisen. Beispielsweise seien genannt Weinsäuremonoglycerid, Weinsäurediglycerid, Citronensäuremonogiycerid, Citronensäurediglycerid, Äpfelsäuremonoglycerid, Äpfelsäurediglycerid sowie deren technische Gemische, die untergeordnet aus dem Herstellungsprozess noch geringe Mengen an Triglycerid enthalten können.

Ebenfalls geeignet sind Anlagerungsprodukte von 1 bis 30, vorzugsweise 5 bis 10 Mol Ethylenoxid an die genannten Partialglyceride.

Als hydrophile Wachse eingesetzt werden können Partialester von Glycerin und/oder Sorbitan -mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen, sofern sie einen Schmelzpunkt von > 30 °C aufweisen.

Sorbitanester

Als Sorbitanester können beispielsweise die folgenden Verbindungen eingesetzt werden, sofern Sie einen Schmelzpunkt von > 30 °C aufweisen.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat. Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Polyglycerinester

Als Polygylcerinester können beispielsweise die folgenden Verbindungen eingesetzt werden, sofern Sie einen Schmelzpunkt von > 30 °C aufweisen:

Typische Beispiele für geeignete Polyglycerinester sind Polyglyceryl-4 Diisostearate/Polyhydroxystearate/ Sebacate, , (Isolan GPS), Polyglyceryl-1 Dipolyhydroxystearate (Dehymuls PGPH), Polyglycerin-3-Diisostearate (Lameform TGI), Polyglyceryl-4 Isostearate (Isolan Gl 34), Polyglyceryl-3 Oleate, Diisostearoyl Polyglyceryl-3 Diisostearate (Isolan PDI), Polyglyceryl-3 Methylglucose Distearate (Tego Care 450), Polyglyceryl-3 Beeswax (Cera Bellina), Polyglyceryl-4 Caprate (Polyglycerol Caprate T2010190), Polyglyceryl-3 Cetyl Ether (Chimexane NL), Polyglyceryl-3 Distearate (Cremophor GS 32) und Polyglyceryl Polyricinoleate (Admul WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter **Fetten** versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar sind sogenannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit $C_{12}$-$C_{60}$-Feftsäuren und insbesondere $C_{12}$-$C_{36}$-Fettsäuren. Hierzu zählt gehärtetes Rizinusöl, ein Dreifachester aus Glycerin und einer Hydroxystearinsäure, der beispielsweise unter der Bezeichnung Cutina® HR im Handel ist. Ebenso geeignet sind Glycerintristearat, Glycerintribehenat (z. B. Syncrowax® HRC), Glycerintripalmitat oder die unter der Bezeichnung Syncrowax® HGLC bekannten Triglycerid-Gemische, mit der Vorgabe, dass der Schmelzpunkt der Wachskomponente bzw. des Gemisches bei 30 °C oder darüber liegt.

Als Wachs sind erfindungsgemäß insbesondere einsetzbar Partialester von C12 bis C22 Fettsäuren mit mehrwertigen Alkoholen, insbesondere mit Glycerin.

Als Wachs sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata® AB und Novata® B (Gemisch aus $C_{12}$-$C_{18}$-Mono-, Di- und Triglyceriden) sowie Cutina® MD oder Cutina® GMS (Glycerylstearat).

Als Wachs einsetzbar sind weiterhin die $C_{12}$-$C_{50}$-Pettalkohole. Geeignet sind insbesondere $C_{12}$-$C_{24}$-Fettalkohole, die auch in Kombination mit den C12-C24 Partialestern von mehrwertigen Alkoholen einsetzbar sind. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertaig, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole®) oder die teilweise verzweigten Alkohole aus der Oxosynthese (Dobanole®) verwendet werden. Erfindungsgemäß besonders bevorzugt geeignet sind $C_{14}$-$C_{22}$-Fettalkohole, die beispielsweise von der Cognis Deutschland GmbH unter der Bezeichnung Lanede® 16 ($C_{16}$-Alkohol), Lanefte® 14 ($C_{14}$-Alkohol), Lanette® O ($C_{16}$/$C_{18}$-Alkohol) und Lanette® 22 ($C_{18}$/$C_{22}$-Alkohol) vermarktet werden. Fettalkohole verleihen den Zusammensetzungen ein trockeneres Hautgefühl als Triglyceride und sind daher gegenüber letzteren bevorzugt.

Als Wachskomponenten können auch $C_{14}$-$C_{40}$-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise **natürliche pflanzliche Wachse,** wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und **tierische Wachse,** wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/ oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die $C_{16}$-$C_{40}$-Alkylstearate, $C_{20}$-$C_{40}$-Alkylstearate (z. B. Kesterwachs® K82H), $C_{20}$-$C_{40}$-Dialkylester von Dimersäuren, $C_{18}$-$C_{38}$-Alkylhydroxystearoylstearate oder $C_{20}$-$C_{40}$-Alkylerucate. Ferner sind $C_{30}$-$C_{50}$-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetearylbehenat und Behenylbehenat einsetzbar.

In einer bevorzugten Ausführungsform der Erfindung enthält die wasserunlösliche Komponente (A) mindestens einen Ölkörper ausgewählt aus der Gruppe bestehend aus Kohlenwasserstoffen, Dialkylether, Dialkylcarbonate, Triglycerid-Mischungen, Ester aus C6-C22-Fettsäuren und C6-C22 Fettalkoholen, Polycarbonate bzw. ein Gemisch dieser Substanzen, Silikonöle und Mischungen hieraus.

Die Menge an wasserunlöslicher Komponenten (A) beträgt weniger als 50 Gew.-%, bevorzugt weniger als 45 Gew.%, insbesondere weniger als 40 Gew.-% bezogen auf das Gesamtgewicht des Emulsionskonzentrates.

Bei der Berechnung der Menge von Komponente (A) werden die hydrophilen nichtionogenen Emulgatoren (B) und die Coemulgatoren (C) nicht berücksichtigt.

Hydrophile nichtionogene Emulgatoren (B)

Als hydrophile nichtionogene Emulgatoren (B) eignen sich bevorzugt Anlagerungsprodukte von Ethylenoxid an lineare Fettalkohole, Fettsäuren, Fettsäurepartialglyceride, Fettsäurepartialpolygylceride, Sorbitan-Fettsäureester oder Alkyl-(oligo)-glycoside mit einem HLB-Wert von 11 -19. Als HLB-Wert soll dabei der Wert gemäß Formel 1

$$HLB = \frac{100 - L}{5} \qquad (I)$$

verstanden werden, in der L den Anteil (in Gew.-%) der lipophilen Alkyl- oder Acylgruppen in den Ethylenoxid-Anlagerungsprodukten bedeutet. Bevorzugt geeignete hydrophile nichtionogene Emulgatoren sind die Anlagerungsprodukte von 8 - 30 Mol Ethylenoxid an lineare Fettalkohole mit 12 - 22 C-Atomen.

Die Emulsionskonzentrate können die Komponenten (B) im Mengen von 1 bis 25, vorzugsweise 5 bis 20, insbesondere 8 bis 12 Gew.% - bezogen auf das Gesamtgewicht des Konzentrats- enthalten.

Lipophile Coemulgatoren (C)

Die lipophilen Coemulgatoren (C) sind nichtionogene, polare Lipidstoffe mit einer oder mehreren Hydroxylgruppen, die in Wasser unlöslich oder nur dispergierbar sind und die aufgrund ihrer niedrigen Hydrophilie allein nicht zur Herstellung von Öl-in-Wasser-Emulsionen geeignet sind.

Als Coemulgatoren sind erfindungsgemäß solche vom Typ der gesättigten Fettalkohole mit 16 - 22 C-Atomen, z.B. Cetylalkohol, Stearylalkohol, Arachidylalkohol oder Behenylalkohol oder Gemische dieser Alkohole geeignet, wie sie bei der technischen Hydrierung von pflanzlichen und tierischen Fettsäuren mit 16 - 22 C-Atomen oder der entsprechenden Fettsäuremethylester erhalten werden. Weiterhin eignen sich als Coemulgatoren Partialester aus einem Polyol mit 3 - 6 C-Atomen und gesättigten Fettsäuren mit 14 - 22 C-Atomen. Solche Partialester sind z.B. die Monoglyceride von Palmitin- und/oder Stearinsäure, die Sorbitanmono- undloder -diester von Myristinsäure, Palmitinsäure, Stearinsäure oder von Mischungen dieser Fettsäuren, die Monoester aus Trimethylolpropan, Erythrit oder Pentaerythrit und gesättigten Fettsäuren mit 14 - 22 C-Atomen. Als Monoester werden auch die technischen Monoester verstanden, die durch Veresterung von 1 Mol Polyol mit 1 Mol Fettsäure erhalten werden und die ein, Gemisch aus Monoester, Diester und unverestertem Polyol darstellen Besonders gut eignen sich gesättigte Fettalkohole mit 16 - 22 C-Atomen oder Partialester von Polyolen mit 3 - 6 C-Atomen und Fettsäuren mit 14 - 22 C-Atomen.

Die Emulsionskonzentrate können die Komponenten (C) im Mengen von Mengen von 1 bis 25, vorzugsweise 5 bis 20, insbesondere 8 bis 15 Gew.% - bezogen auf das Gesamtgewicht des Konzentrats- enthalten.

Polyole (D)

Als Polyole werden mehrwertige Alkohole, d.h. organische Verbindungen, die mindestens 2 alkoholische Hydroxylgruppen im Molekül tragen bezeichnet. In einer Ausführungsform der Erfindung enthalten die Polyole 2 bis 6 Hydroxylgruppen pro Molekül. In einer Ausführungsform der Erfindung werden als Polyole niedermolekulare mehrwertige Alkohole eingesetzt, d.h. Verbindungen die 2 bis 18, insbesondere 2 bis 10, vorzugsweise 2 bis 6 C-Atome enthalten.

In einer bevorzugten Ausführungsform der Erfindung werden als Polyole (D) Verbindungen eingesetzt, die mindestens 2 Hydroxylgruppen pro Molekül tragen und aus 2 bis 18, vorzugsweise 2 bis 10, insbesondere aus 2 bis 6 C-Atomen bestehen.

In einer bevorzugten Ausführungsform der Erfindung werden als Polyole (D) Verbindungen eingesetzt, die 2 bis 6 Hydroxylgruppen pro Molekül tragen und aus 2 bis 6 C-Atomen bestehen.

Als Polyole (D) können sowohl einzelne Polyole als auch Mischungen aus beliebigen Polyolen eingesetzt werden. In einer bevorzugten Ausführungsform werden als Polyole Mischungen aus mindestens 2, insbesondere mindestens 3 Polyolen eingesetzt.

Die Polyole können noch weitere funktionelle Gruppen, insbesondere Aminogruppen, enthalten bzw. mit Stickstoff modifiziert sein. In einer bevorzugten Ausführungsform enthalten die Polyole außer den Hydroxylgruppen keine weiteren funktionellen Gruppen.

Typische Beispiele für erfindungsgemäß einzusetzende Polyole sind :

- Glycerin, Diglycerin, Triglycerin, Tetraglycerin Alkylenglycole, wie beispielsweise Ethylenglycol, Diethylenglycol, Triethylenglykol, 1,2-Propylenglycol, 1,3-Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- kurzkettige Alkyglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Erythrit, Arabit, Adonit (Synonym Ribit), Xylit, Sorbit, Mannit und Dulcit (Synonym Galactit).
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

In einer bevorzugten Ausführungsform der Erfindung wird als Polyol (D) mindestesn eine Verbindung eingesetzt, ausgewählt aus der Gruppe bestehend aus Glycerin, 1,2-Propylenglykol, Sorbit, Butylenglykol und Hexylenglykol.

Die Emulsionskonzentrate können die Komponenten (D) im Mengen von 1 bis 25, vorzugsweise 5 bis 20, insbesondere 8 bis 15 Gew.% - bezogen auf das Gesamtgewicht des Konzentrats- enthalten.

Wässriger Anteil

In einer bevorzugten Ausführungsform der Erfindung enthalten die erfindungsgemäßen Emulsionskonzentrate 35 bis 55 Gew.-% wässrigen Anteil bezogen auf das Gesamtgewicht des Emulsionskonzentrates.

Weitere Bestandteile

Die erfindungsgemäßen Emulsionskonzentrate können weitere Bestandteile enthalten, wie beispielsweise Konservierungsmittel, biogene Wirkstoffe, UV-Lichtschutzfilter, Verdickungsmittel, Überfettungsmittel, Stabilisatoren, Polymere, Antioxidantien, Deodorantien, Filmbildner, Quellmittel, Insektenrepellentien, Hydrotrope, Solubilisatoren, Parfümöle, Farbstoffe etc. Die Mengen der jeweiligen Zusätze richten sich nach der beabsichtigten Verwendung.

Sofern weitere Bestandteile unter die Definition der wasserunlöslichen Ölkomponenten (A) fallen, werden sie bei der Berechnung der Verhältnisse von (A) : (B) : (C) : (D) berücksichtigt, indem sie zur Komponenten (A) hinzugezählt werden. Dies gilt für alle weiteren Bestandteile, ist aber insbesondere für lipophile UV-Lichtschutzfiltem und Parfümöle relevant.

Die erfindungsgemäßen Emulsionskonzentrate enthalten die weiteren Bestandteile üblicherweise in Mengen von < 25 Gew.%, insbesondere < 20 Gew.% bezogen auf die Gesamtmenge des Konzentrates.

Als **Konservierungsmittel** eignen sich beispielsweise Phenoxyethanol, Formaldehydlösung, Parabene, Mischungen aus Phenoxyethanol und Ethylhexylglycerin (wie sie beispielsweise unter dem Handelsnamen Euxyl PE 9010 erhältlich sind) oder Sorbinsäure sowie die unter der Bezeichnung Surfacine® bekannten Silberkomplexe und die in Anlage 6, Teil A und B der Kosmetikverordnung aufgeführten weiteren Stoffklassen.

In einer bevorzugten Ausführungsform der Erfindung wird das Konservierungsmittel ausgewählt aus der Gruppe bestehend aus Phenoxyethanol, Formaldehydlösung Parabene; organische Säuren und Mischungen daraus, gegebenenfalls in Kombination mit Pentandiol und/oder Ethylhexylglycerin.

In einer Ausführungsform der Erfindung enthaltend die erfindungsgemäßen Emulsionskonzentrate als weiteren Bestandteil mindestens ein UV.Lichtschutzfilter.

Erfindungsgemäß sind als UV-Lichtschutzfaktoren bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:

➣ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben;
➣ 3-(4'-Trimethylammonium) benzyliden- bornan-2-on-methylsulfat (Mexoryl SO)
➣ 3,3'-(1,4-Phenylendimethin)-bis (7,7- dimethyl-2-oxobicyclo-[2.2.1] heptan-1-methansulfonsäure) and salts (Mexoryl SX)
➣ 3-(4'-Sulfo)-benzyliden-bornan-2-on and salts (Mexoryl SL)
➣ Polymer von N-{(2und 4)- [2-oxoborn-3-yliden)methyl}benzyl]acrylamid(Mexoryl SW)
➣ 2-(2H-Benzomazol-2-yl)-4-methyl-6-(2-methyl-3-(1,3,3,3-tetramethyl-1-(trimethylsilyloxy) disiloxanyl)propyl) phenol (Mexoryl XL)
➣ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➣ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
➣ Ester der Salicylsäure, vorzugsweise Salicylsäure-2-ethylhexylester, Salicylsäure-4-iso-propylbenzylester, Salicylsäurehomomenthylester;
➣ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➣ Ester der Benzalmalonsäure, vorzugsweise 4-Methoxybenzmalonsäuredi-2-ethylhexyl-ester;
➣ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4-diyl)dümino] bis(benzoesäure-2- ethylhexylester) (Uvasorb® HEB);
➣ 2,2(-Methylen-bis(6-(2H-benzotriazol-2-yl)-4-(1,1,3,3-tetramethyl-butyl)phenol) (Tinosorb M);
➣ 2,4-Bis[4-(2-ethylhexyloxy)-2- hydroxyphenyl]-6-(4- methoxyphenyl)-1,3,5- triazin (Tinosorb S);
➣ Propan-1,3-dione, wie z.B. 1-(4-tert.Butytphenyl)-3-(4'methoxyphenyt)propan-1,3-dion:
➣ Ketotricyclo(5.2.1.0)decan-Derivate, wie in der EP 0694521 B1 beschrieben;
➣ Dimethicodiethylbenzaimalonate (Parsol SLX).

Als wasserlösliche UV - Filter kommen in Frage: -

➣ 2-Phenylbenzimidazol-5-sulfonsäure und deren Alkali-, Erdalkali-, Ammonium-, Alkylammonium-, Alkanolammonium- und Glucammoniumsalze;
➣ 2,2(-(1,4-Phenylen)bis(1H-benzimidazol-4,6-disulfon-säure, Mononatriumsalz) (Neo Heliopan AP)
➣ Sulfonsäurederivate von Benzophenonen, vorzugsweise 2-Hydroxy-4-methoxybenzo-phenon-5-sulfonsäure und ihre Salze;
➣ Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4-tert.Butylphenyl)-3-(4'-methoxyphenyl)propan-1,3-dion 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Erfindungsgemäß bevorzugt sind UV-Lichtschutzfilter ausgewählt aus dem Anhang VII der europäischen Kosmetik-Gesetzgebung (24th Adapting Comission Directive, 29.Februar 2000).

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Typische Beispiele sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Weitere geeignete UV-Lichtschutzfilter sind der Übersicht von P.Finkel in SÖFW-Journal 122, 543 (1996) sowie Parf.Kosm. 3, 11 (1999) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin; Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Camosin, Butylhydroxytoluol, Butylhydroxyanisol, Nordihydroguajakharzsäure, Nordihydro-guajaretsäure, Trihydroxybutyrophenon, Harnsäure und deren Derivate, Man-nose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stil-benoxid, trans-Stil-benoxid) und die erfindungsgemäß geeigneten Derivate (Salze, Ester, Ether, Zucker, Nukleotide, Nukleoside, Peptide und Lipide) dieser genannten Wirkstoffe.

In einer Ausführungsform der Erfindung enthaltend die erfindungsgemäßen Emulsionskonzentrate als weiteren Bestandteil mindestens einen **biogenen Wirkstoff**.

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.

B. Prunusextrakt, Bambaranussextrakt und Vitaminkomplexe zu verstehen.

In einer bevorzugten Ausführungsform der Erfindung enthaltend die erfindungsgemäßen Dispersionen als biogenen Wirkstoff mindestens eine Verbindung ausgewählt aus Vitaminen, Allantoin, Bisabolol und Pflanzenextrakten.

In einer bevorzugten Ausführungsform der Erfindung enthaltend die erfindungsgemäßen Dispersionen als biogenen Wirkstoff mindestens eine Verbindung ausgewählt aus Tocopherol, Tocopherolacetat, Tocopherolpalmitat, Ascorbinsäure, β-Glucane, Retinol, Bisabolol, Allantoin, Phytantriol, Panthenol, AHA-Säuren, Pflanzenextrakten und Mischungen daraus.

In einer Ausführungsform der Erfindung enthaltend die erfindungsgemäßen Emulsionskonzentrate als weiteren Bestandteil mindestens ein **Verdickungsmittel.**

Als **Verdickungsmittel** eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox),

In einer Ausführungsform der Erfindung enthaltend die erfindungsgemäßen Emulsionskonzentrate als weiteren Bestandteil mindestens ein **desodorierenden Wirkstoff.**

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindem und Anwendung in Depigmentierungsmitteln finden, kommen beispielsweise Arbutin, Ferulasäure, Kojisäure, Cumarinsäure und Ascorbinsäure (Vitamin C) in Frage.

Als **Farbstoffe** können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I. 75810), Chinolingelb (C.I.47005). Titandioxid (C.I.77891), Indanthrenblau RS (C.I. 69800) und Krapplack (C.I.58000). Diese Farbstoffe werden üblicherweise in Konzentrationen von 0,001 bis 0,1 Gew.%, bezogen auf die gesamte Mischung, eingesetzt.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage

Weitere Bestandteile der Emulsionskonzentrate (wie z.B. Konservierungsmittel, kosmetische Wirkstoffe, UV-Filter etc.) werden je nach ihrer Löslichkeit entweder über die Wasserphase oder über die lipophilen Phase zu gegeben.

Herstellung der erfindungsgemäßen Emulsionskonzentrate

Die Herstellung der erfindungsgemäßen Emulsionskonzentrate erfolgt bevorzugt nach dem in EP 0723432 B1 beschriebenen Verfahren. Es ist daher bevorzugt, die Ölkomponente (A), den hydrophilen Emulgator (B) und den lipophilen Coemulgator (C) so auszuwählen, dass die damit hergestellten Emulsionen eine Phaseninversionstemperatur im Bereich unter 100°C aufweisen. Die Emulgatoren (B) und Coemulgatoren (C) werden mit der Ölkomponente (A) gemischt und bis 5 °C über die Phaseninversionstemperatur erwärmt. Dann werden Wasser und Polyol (D) von etwa gleicher Temperatur unter Rühren zugesetzt - oder umgekehrt, die Mischung von Ölkomponente, Emulgator und Coemulgator in das auf 5 °C über Phaseninversionstemperatur erwärmte Wasser mit Polyol eingerührt. Alternativ kann die Emulgierung auch unterhalb der Phaseninversionstemperatur durchgeführt werden und die Emulsion dann bis 5°C über die Phaseninversionstemperatur gebracht werden. Nach dem Abkühlen erhält man dann ein sehr feinteiliges Emulsionskonzentrat.

Die :Herstellung der Emulsionskonzentrate- erfolgt vorzugsweise nach der so genannten Phasen-Inversions-Methode (PIT). Die PIT Technologie ist im Stand der Technik beschrieben.

Die erfindungsgemäßen Emulsionskonzentrate weisen üblicherweise eine Viskosität von größer gleich 200 mPas, bevorzugt größer gleich 400 mPas auf (gemessen mit einem Brookfield Rotationsviskosimeter Typ RVF, Spindel 4,10 UpM, 20°C).

Verwendung der Emulsionskonzentrate

Die erfindungemäßen Emulsionskonzentrate können direkt als kosmetische oder pharmazeutische Zubereitung verwendet werden.

Ein weiterer Gegenstand der Erfindung betrifft daher die Verwendung der erfindungsgemäßen Emulsionskonzentrate als kosmetische oder pharmazeutische Zubereitungen.

Die erfindungsgemäßen Emulsionskonzentrate sind feinteilig und sehr lagerstabil, sie eignen sich daher ganz besonders als vorgefertigter Emulsionsbaustein, der aufgrund seiner Fließfähigkeit gut für die Lagerung und den Transport an einen Verarbeitungsort mit geringer technischer Ausrüstung geeignet ist, um dort mit einfachsten Mitteln brauchbare kosmetische und pharmazeutische Emulsionen, insbesondere Öl-in-Wasser Emulsionen herzustellen.

Ein Gegenstand der Erfindung betrifft demnach die Verwendung der erfindungsgemäßen Emulsionskonzentrate zur Herstellung von kosmetischen oder pharmazeutischen Zubereitungen.

Das erfindungsgemäße Emulsionskonzentrat eignet sich insbesondere zur Herstellung kosmetischer oder pharmazeutischer Öl-in-Wasser-Emulsionen, Dabei kann entweder die kontinuierliche wässrige Phase oder die lipophile Phase oder beides ohne weitere Wärmezufuhr in das Emulsionskonzentrat eingearbeitet werden.

Die wässrige Phase, mit der das Emulsionskonzentrat verdünnt wird, kann beliebige wasserlösliche Bestandteile, z.B. wasserlösliche kosmetische Wirkstoffe, wasserlösliche Proteine oder Proteinabbauprodukte, Konservierungsmittel, Farbstoffe, Duftstoffe, Magnesiumsalze oder andere übliche wasserlösliche Komponenten gelöst enthalten. Bevorzugt enthält die wässrige, kontinuierliche Phase ein wasserlösliches, natürliches oder synthetisches Polymerisat, das die kosmetischen Eigenschaften der Emulsionen durch Erhöhung der Viskosität verbessert. Eine besonders wirksame Kombination von Hydrocolloiden zur Verbesserung der kosmetischen Eigenschaften solcher Emulsionen ist ein Gemisch aus nichtionischen Celluloseethem, z.B. Hydroxypropylcellulose und vernetzten Acrylsäure-Polymerisaten, wie sie z.B. unter der Handelsbezeichnung Carbopol[(R)] erhältlich sind (vgl. DE 3521713 A1).

Die liphophile Phase, mit der das Emulsionskonzentrat verdünnt wird, kann beliebige lipophile Bestandteile, z.B. liphophile kosmetische Wirkstoffe, enthalten. Als lipophile Phase eignen sich alle als Komponente (A) geeigneten Verbindungen;

In der Regel werden 1 bis 50, bevorzugt 2 bis 30, insbesondere 4 bis 10 Gew.-% des Emulsionskonzentrates für die Herstellung der kosmetischen oder pharmazeutischen Zubereitungen eingesetzt.

Kosmetische Zubereitungen

Ein weiterer Gegenstand der Erfindung betrifft kosmetische oder pharmazeutische Zubereitungen, welche wasserunlösliche Ölkomponenten (A), hydrophile nichtionische Emulgatoren (B), lipophile Coemulgatoren (C), Polyole (D) im Gewichtsverhältnis A : B : C : D =1: (0,25 - 0,6) : (0,25 - 0,6) : (0,45 - 0,65) enthält.

Überraschenderweise wurde gefunden, dass durch das erfindungsgemäße Verhältnis der Komponenten zueinander stabile, feinteilige Zubereitungen, insbesondere Öl-in-Wasser Emulsionen erhalten werden können.

Die erfindungsgemäßen Zubereitungen können beispielsweise hergestellt werden, indem man die erfindungsgemäßen Emulsionskonzentrate entsprechend verdünnt. Im einfachsten Fall erfolgt die Verdünnung mit Wasser, da so das erfindungsgemäße Verhältnis der Komponenten nicht verändert wird. Ebenso ist es aber auch möglich die erfindungsgemäßen Zubereitungen durch dem Fachmann bekannte Verfahren herzustellen, wie z.B. einfaches Mischen der Komponenten (A) bis (D) und gegebenenfalls Wasser oder durch bekannte Verfahren der Emulsionsherstellung.

Verwendung zur Beschichtung von Substraten

Die erfindungsgemäßen Emulsionskonzentrate sowie die erfindungsgemäßen kosmetischen oder pharmazeutischen Zubereitungen eignen sich insbesondere zur Beschichtung von Substraten.

Die Emulsionskonzentrate sowie die Zubereitungen sind besonders geeignet zur Applikation auf Papieren, Tüchem, Textilien und Watteprodukten, die im Bereich der Babypflege und -hygiene sowie im Bereich der Make-up-Entfemung, insbesondere der Augen-Make-up-Entfemung, im Bereich der Damenhygiene (Tampons, Damenbinden, Slip-Einlagen) und im Bereich der Körperhygiene (Toilettenpapier, Feuchttoilettenpapier) eingesetzt werden.

Ein weiter Gegenstand der Anmeldung ist daher die Verwendung der erfindungsgemäßen Emulsionskonzentrate oder der erfindungsgemäßen Zubereitungen auf Papieren, Vliese (Nonwoven) und Geweben (Woven). Erfindungsgemäß zählen hierzu alle Papierarten, Vliese und Gewebe, die dem Fachmann geläufig sind, und Produkte, die daraus herstellbar sind, wie z.B. Toilettenpapier, Papiertaschentücher, Tissues, Wipes, Watte, Wattepads, Make-up Entferner, Tampons, Binden, Slip-Einlagen, Windeln, Babypflegetücher, Babyreinigungstücher, Textilien, etc. Ebenso Gegenstand der Erfindung sind Papier-, Vlies- und Gewebe-Produkte zur Körperpflege und - reinigung, welche wasserunlösliche Ölkomponenten (A), hydrophile nichtionische Emulgatoren (B), lipophile Coemulgatoren (C), Polyote (D) im Gewichtsverhältnis A : B : C : D =1: (0,25 - 0,6) : (0,25 - 0,6) : (0,45 - 0,65) enthält.

Als Substrate dieser Papier-, Vlies- und Gewebe-Produkte seinen exemplarisch genannt: Träger aus Textilfaser, z.B. aus Naturfaser, wie Cellulose, Seide, Wolle, Regeneratcellulose (Viskose, Rayon), Cellulosederivate und/oder synthetische Fasern, wie z.B.Polyester-, Polypropylen-, Polyethylenterephtalat-, Polyamin-, Polyolifin-, Polyacrylnitril-Fasern oder Mischungen solcher Fasern, gewebt oder ungewebt.

Die erfindungsgemäßen Produkte können nach dem Fachmann bekannten Verfahren hergestellt werden. Der Auftrag der erfindungsgemäßen Emulsionskonzentrate oder der erfindungsgemäßen Zubereitungen auf die erfindungsgemäßen Papier-, Vlies- und Gewebe-Produkte zur Körperpflege und -reinigung erfolgt dabei nach dem Fachmann bekannten Methoden, wie beispielsweise Imprägnieren, Tränken, Eintauchen, Ansprühen, Abstreifen oder Beschichten. Dies kann sowohl bei Raumtemperatur als auch bei erhöhten Temperaturen erfolgen.

Die erfindungsgemäßen Emulsionskonzentrate oder die erfindungsgemäßen Zubereitungen können vor dem Auftrag auf das Papier-, Vlies- und Gewebe-Substrat verdünnt werden und gegebenenfalls kann das erhaltene .Papier-, Vlies- und Gewebe-Produkt anschtießend getrocknet werden

### Beispiele

Die folgenden Beispiele-sollen die Erfindung näher erläutern:

Herstellung von Emulsionskonzentraten:

Die hydrophilen Emulgatoren (B) und lipophilen Coemulgatoren (C) wurden zusammen mit der Ölkomponente (A) bis auf 95 °C erwärmt. Dann wurden das Wasser und das Polyol (D) mit gleicher Temperatur hinzu gegeben und intensiv gemischt. Nach dem Abkühlen auf 20°C wurde eine feinteilige Emulsion erhalten. Die Viskositätsmessung erfolgte jeweils 5 Stunden nach Herstellung der Emulsion mit Hilfe eines Rotationsviskosimeters.

Tabelle 1 zeigt die Zusammensetzung der erfindungsgemäßen Beispiele sowie die Ergebnisse der Stabilitätsmessungen. In Tabelle 2 sind die Verhältnisse der Komponenten (A), (B), (C) und (D) aufgeführt.

Nur die erfindungsgemäßen Emulsionskonzentrate sind bei Lagerung stabil.

**Tabelle 1:** Emulsionskonzentrate (alle Angaben in Gew.% bezogen auf das Gesamtgewicht des Konzentrates):

| Komponente (INCI) | | 1 | 2 | 3 | 4 | 5 | 6 | V1 |
|---|---|---|---|---|---|---|---|---|
| (B) | Ceteareth-12 | 1,0 | 3,0 | 2,0 | 1,5 | 1,5 | 1,0 | 1,0 |
| (B) | Ceteareth-20 | 9,0 | 6,0 | 10,0 | 8,0 | 8,0 | 8,0 | 6,5 |
| (C) | Cetearyl Alcohol | 7,0 | 4,0 | 5,5 | 4,0 | 5,0 | 4,5 | 7,0 |
| (C) | Glyceryl Stearate | 2,0 | 5,0 | 5,5 | 6,0 | 5,0 | 4,5 | 7,0 |
| (A) | Cetearyl Isononanoate | 18,0 | 5,0 | | 14,0 | | 25,0 | 18,0 |
| (A) | Coco Caprylate Caprate | | 15,0 | 11,0 | | 12,0 | | |
| (A) | Paraffinum Perliquidum | | | | 5,0 | | | |
| (A) | Caprylic / Capric Triglyceride | | | | | 6,0 | | |
| (A) | Isopropyl Palmitate | | | 11,0 | | | | |
| (D) | Glycerin | 10,0 | 12,0 | 10,5 | 10,0 | 11,0 | 11,5 | 5,0 |
| | Wasser | 53,0 | 50,0 | 44,5 | 51,5 | 51,5 | 45,5 | 55,5 |
| | pH-Adjuster; Konservierungsmittel | pH 3,0 -3,3; q.s. | | | | | | |
| | | | | | | | | |
| | Stabilitätsdaten | 3 Monate 40°C stabil | | | | | | Separation < 1 Woche |

(fortgesetzt)

| Komponente (INCI) | | 1 | 2 | 3 | 4 | 5 | 6 | V1 |
|---|---|---|---|---|---|---|---|---|
| | Viskosität (RT) (Brookfield RVF, Spindel 4, 10 UpM), [mPa*s] | 600 | 800 | 900 | 1800 | 2000 | 2500 | - |
| | Viskosität (40°C) (Brookfield RVF, Spindel 4, 10 UpM), [mPa*s] | 2500 | 1800 | 1700 | 2100 | 2300 | 2800 | - |
| | Teilchengröße (Coufter LS) [nm] | 105 | 110 | 95 | 115 | 110 | 120 | 500 |

**Tabelle 2:**

| Verhältnis (A) : (B) : (C) : (D) | |
|---|---|
| Beispiel 1 | 1: 0,56 : 0,50 : 0,56 |
| Beispiel 2 | 1: 0,45 : 0,45 : 0,6 |
| Beispiel 3 | 1: 0,54 : 0,50 : 0,48 |
| Beispiel 4 | 1: 0,50 : 0,53 : 0,53 |
| Beispiel 5 | 1: 0,53 : 0,55 : 0,61 |
| Beispiel 6 | 1: 0,36 : 0,36 : 0,46 |
| Vergleichsbeispiel V1 | 1: 0,42 : 0,77 : 0,28 |

**Patentansprüche**

1. Emulsionskonzentrat mit einem Gehalt an wasserunlöslichen Ölkomponenten (A), hydrophilen nichtionischen Emulgatoren (B), lipophilen Coemulgatoren (C), Polyolen (D) und Wasser, welches

   a. weniger als 50 Gew.% wasserunlösliche Ölkomponente (A) enthält

   - bezogen auf das Gesamtgewicht des Konzentrats -

   b. die Komponenten (A), (B) und (C) und (D) im Gewichtsverhältnis A : B : C : D = 1: (0,25 - 0,6) : (0,25 - 0,6) : (0,45 - 0,65) enthält.

2. Emulsionskonzentrat nach Anspruch 1, **dadurch gekennzeichnet, dass** es einen wässrigen Anteil von 35 bis 55 Gew.% enthält.

3. Emulsionskonzentrat nach mindestens einem der Ansprüche 1 bis 2, **dadurch gekennzeichnet, dass** die wasserunlösliche Ölkomponente (A) ausgewählt ist aus bei 30°C flüssigen Kohlenwasserstoffen, Dialkylethern, Fettsäureestern mit 12 - 44 C-Atomen, Dialkylcarbonate, Guerbetalkohole und Siliconölen oder deren Mischungen.

4. Emulsionskonzentrat nach mindestens einem der Ansprüche 1 bis 3, **dadurch gekennzeichnet, dass** mindestens 60 Gew. %.der wasserunlöslichen Ölkomponente (A) eine mittlere Polarität von größer gleich 20 mN/m und kleiner gleich 30 mN/m aufweisen.

5. Emulsionskonzentrat nach mindestens einem der Ansprüche 1 bis 4, **dadurch gekennzeichnet, dass** als hydrophile Emulgatoren (B) Anlagerungsprodukte von Ethylenoxid an lineare Fettalkohole, Fettsäuren, Fettsäurepartialglyceride, Sorbitan-Fettsäureester oder Alkylglycoside mit einem HLB-Wert von 11 -19 enthalten sind.

**6.** Emulsionskonzentrat nach mindestens einem der Ansprüche 1 bis 5, **dadurch gekennzeichnet, dass** als lipophile Coemulgatoren (C) gesättigte Fettalkohole mit 12 - 22 C-Atomen oder Partialester von Polyolen mit 3 - 6 C-Atomen und Fettsäuren mit 12 - 22 C-Atomen oder Gemischen davon enthalten sind.

**7.** Emulsionskonzentrat nach mindestens einem der Ansprüche 1 bis 6, **dadurch gekennzeichnet, dass** als Polyole niedermolekulare mehrwertige Alkohole enthalten sind, die 2 bis 18 C-Atome enthalten.

**8.** Emulsionskonzentrat nach Anspruch 7, **dadurch gekennzeichnet, dass** die Polyole 2-bis 6 Hydroxylgruppen tragen.

**9.** Emulsionskonzentrat nach Anspruch 7, **dadurch gekennzeichnet, dass** als Polyole mindestens eine Verbindung eingesetzt wird, ausgewählt aus der Gruppe bestehend aus Glycerin, 1,2-Propylenglykol, Sorbit, Butylenglykol und Hexylenglykol.

**10.** Verwendung der Emulsionskonzentrate nach mindestens einem der Ansprüche 1 bis 9 als kosmetische oder pharmazeutische Zubereitung.

**11.** Verwendung der Emulsionkonzentrate nach mindestens einem der Ansprüche 1 bis 9 zur Herstellung kosmetischer oder pharmazeutischer Zubereitungen, insbesondere Öl-in-Wasser-Emulsionen.

**12.** Kosmetische oder pharmazeutische Zubereitung, welche wasserunlösliche Ölkomponenten (A), hydrophile nichtionische Emulgatoren (B), lipophile Coemulgatoren (C), Polyole (D) im Gewichtsverhältnis A : B : C : D =1: (0,25 - 0,6) : (0,25 - 0,6) : (0,45 - 0,65) enthält.

**13.** Verwendung der Emulsionskonzentrate nach mindestens einem der Ansprüche 1 bis 9 oder der Zubereitungen nach Anspruch 12 zur Beschichtung von Substraten

**14.** Papier-, Vlies- oder Gewebe-Produkt zur Körperpflege und -reinigung, welches wasserunlösliche Ölkomponenten (A), hydrophile nichtionische Emulgatoren (B), lipophile Coemulgatoren (C), Polyole (D) im Gewichtsverhältnis A : B : C : D =1: (0,25 - 0,6) : (0,25 - 0,6) : (0,45 - 0,65) enthält.

**Europäisches**
**Patentamt**

**EUROPÄISCHER RECHERCHENBERICHT**

Nummer der Anmeldung

EP 06 01 6953

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 92/07543 A (HENKEL KGAA [DE]) 14. Mai 1992 (1992-05-14) * Beispiele B1,B2,B4-B8 * * Seite 10 - Seite 11 * ----- | 1-14 | INV. A61K8/86 A61K8/31 A61K8/37 A61K8/34 |
| A,D | EP 0 723 432 B1 (HENKEL KGAA [DE]) 30. Dezember 1998 (1998-12-30) * Seite 2, Zeile 18 - Zeile 23 * * Beispiele * ----- | 1-14 | A61K8/02 A61Q19/00 A61K8/06 A61K9/107 |
| A | EP 1 380 279 A (BEIERSDORF AG [DE]) 14. Januar 2004 (2004-01-14) * Beispiele * ----- | 1-14 | |
| A | EP 1 106 170 A (BEIERSDORF AG [DE]) 13. Juni 2001 (2001-06-13) * Beispiele * ----- | 1-14 | |

RECHERCHIERTE
SACHGEBIETE (IPC)

A61K
A61Q

Der vorliegende Recherchenbericht wurde für alle Patentansprüche erstellt

| Recherchenort | Abschlußdatum der Recherche | Prüfer |
|---|---|---|
| München | 17. Januar 2007 | Ruckebusch, Virginie |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
  Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 06 01 6953

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-01-2007

| Im Recherchenbericht angeführtes Patentdokument | Datum der Veröffentlichung | Mitglied(er) der Patentfamilie | Datum der Veröffentlichung |
|---|---|---|---|
| WO 9207543 A | 14-05-1992 | CA 2094935 A1<br>DE 4033928 A1<br>EP 0554292 A1<br>ES 2066481 T3<br>JP 3043410 B2<br>JP 6501942 T<br>US 5494938 A | 26-04-1992<br>30-04-1992<br>11-08-1993<br>01-03-1995<br>22-05-2000<br>03-03-1994<br>27-02-1996 |
| EP 0723432 B1 | 30-12-1998 | AT 175107 T<br>DE 4335045 A1<br>WO 9510259 A1<br>EP 0723432 A1<br>ES 2127417 T3<br>JP 9503767 T<br>JP 3652367 B2<br>US 5817254 A | 15-01-1999<br>20-04-1995<br>20-04-1995<br>31-07-1996<br>16-04-1999<br>15-04-1997<br>25-05-2005<br>06-10-1998 |
| EP 1380279 A | 14-01-2004 | DE 10230061 A1 | 22-01-2004 |
| EP 1106170 A | 13-06-2001 | DE 19957761 A1<br>US 6805871 B1 | 07-06-2001<br>19-10-2004 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0723432 B1
- US 20060013783 A1
- WO 031041676 A
- EP 0693471 B1
- EP 0818450 A1
- EP 0694521 B1
- DE 19712033 A1
- DE 3521713 A1

**In der Beschreibung aufgeführte Nicht-Patentliteratur**

- **P.FINKEL.** *SÖFW-Journal,* 1996, vol. 122, 543
- *Parf.Kosm.,* 1999, vol. 3, 11